# EUROPEAN PATENT APPLICATION

(11) **EP 0 788 824 A2**
(43) Date of publication of application: **13.08.1997**
(21) Application number: 97300751.1
(22) Date of filing: 06.02.1997
(51) Int. Cl.: B01D 29/23

(54) **Filter and method of manufacture**

(30) Priority: 06.02.1996 US 595875; 23.04.1996 US 636242
(71) Applicant: Filtertek, Inc., Hebron, Illinois 60034 (US)
(72) Inventor: Ruschke, Ricky R., McHenry, Illinois 60050 (US); Leahey, John A., Woodstock, Illinois 60098 (US)
(74) Representative: Bayliss, Geoffrey Cyril

(57) **Abstract**

The invention provides a filter (10), such as an integrated blood filter and drip chamber device, which is efficient and economical to manufacture. The preferred filter (10) comprises a housing (20) and a insertion member (40). The housing (20) is comprised of an outlet port (36), an interior surface (22), and a open end (26). The insertion member (40) is comprised of end cap (44), an inlet port (56), and a filter member (42). The end cap (44) mates with the open end (26) of the housing (20) to form an interior chamber (28) within the housing (20). The filter member (42), which is comprised of a filter media (62) supported by a frame (50), comprises an edge which mates with the interior surface of the housing (20), thereby dividing the interior chamber (28) into a first volume (12) in fluid communication with the inlet port (56) and a second volume (14) in fluid communication with the outlet port (36). The filter media (62) is capable of allowing fluid to flow between the first and second volumes (12,14) through the filter media (62) while preventing fluid contaminants from passing between the first and second volumes (12,14).

## Description

### BACKGROUND OF THE INVENTION

Blood filters and drip chambers are well known in the art. However, such devices have been expensive and difficult to manufacture. For example, the typical blood filter is assembled from four or more separate parts requiring three or more separate bonding operations to assemble. In typical blood filters, solvent bonds are used. Solvent bonds are expensive and time consuming to make. Solvent bonds also lack consistency, leading to fluid leakage and failures. A blood filter requiring fewer bonding operations would therefore be a significant improvement in the art.

### SUMMARY OF THE INVENTION

The invention provides a filter, such as an integrated blood filter and drip chamber device, which is efficient and economical to manufacture.

The preferred filter comprises a housing and a insertion member. The housing is comprised of a second port, an interior surface, and a open end. The insertion member is comprised of a first port, a end cap, and a filter member, said filter member comprising a filter media supported by a frame. The end cap mates with the open end of the housing to form an interior chamber within the housing. The filter member comprises an edge which mates with the interior surface of the housing, thereby dividing the interior chamber into a first volume in fluid communication with the first port and a second volume in fluid communication with the second port. The filter media is capable of allowing fluid to flow between the first and second volumes through the filter media while preventing fluid contaminants from passing between the first and second volumes through the filter media.

The preferred filter operates as follows. The fluid to be filtered passes through the first port and enters the first volume. The fluid then passes through the filter media and into the second volume. The filter media prevents any contaminants within the fluid from passing through the filter media and into the second volume. The filtered fluid then exits from the second volume by passing through the second port.

The method of manufacturing the filter is comprised of the following steps. An insertion member having an end cap, an inlet port on the end cap, and a filter member having an edge, the filter member comprising a filter frame and a filter media, is formed by 1) forming the filter media into the shape of a sleeve; 2) placing the filter media over a core pin; 3) inserting the core pin into a cavity mold; and 4) injecting plastic into the cavity mold to form a monolithic member comprising the end cap, inlet port, and the filter frame supporting the filter media. A monolithic housing having an outlet port, an interior surface, and an open end is formed by an injection molding process. The insertion member is then assembled to the housing by bonding the end cap of the insertion member to the open end of the housing. The edge of the filter member is then sealed to the interior surface of the housing with a tongue and groove joint.

The above device and method of manufacture reduces the number of molded parts to two. The device and method of manufacture also reduces the number of solvent bonds to no more than two and as few as one. This reduction in the number of solvent bonds greatly reduces the cost of manufacture and improves the integrity of the filter device.

### BRIEF DESCRIPTION OF THE DRAWINGS

FIG. 1 is a sectional view of the preferred integrated blood filter and drip chamber device.

FIG. 2 is an exploded sectional view of the integrated blood filter and drip chamber device of FIG. 1.

FIG. 3 is a cross-sectional view taken along line 3-3 of FIG. 1.

FIG. 4 is a sectional view of an alternative embodiment of the integrated blood filter and drip chamber device.

### DETAILED DESCRIPTION OF THE DRAWINGS AND PREFERRED EMBODIMENTS OF THE INVENTION

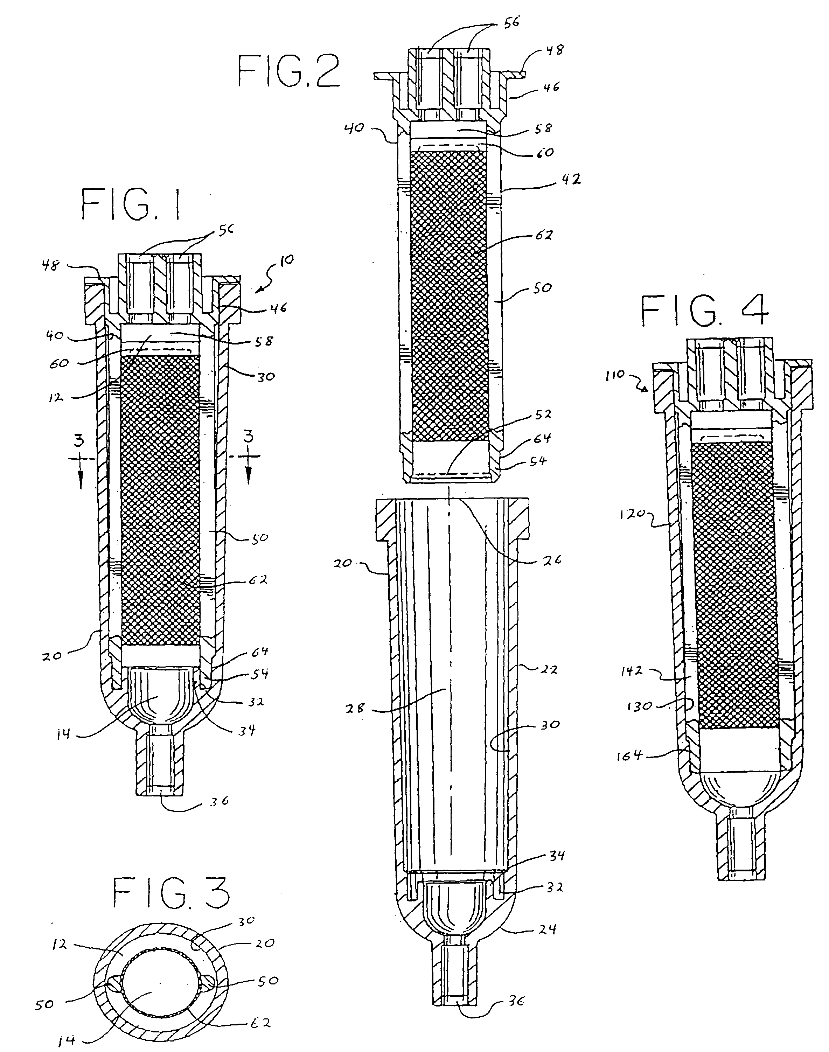

Referring to FIGS. 1-3 of the drawings, reference numeral 10 indicates in general the preferred integrated blood filter and drip chamber device of the present invention. The integrated blood filter and drip chamber device 10 is comprised of a housing 20 and a insertion member 40.

In the preferred embodiment shown, the housing 20 is comprised of an interior chamber 28 formed by a cylindrical side wall 22, a closed end 24, and an open end 26. A second port 36, preferably an outlet port comprising a tubing connector, is provided in the closed end 24 of the housing 20 for the delivery of fluids from the interior chamber 28. The housing 20 also comprises a groove 32 formed by a lip 34 which projects inwardly from the interior surface 30 of the housing 20. In the preferred embodiment shown, the surface of the closed end 24 is curved and the groove 32 is formed extending inwardly from the curved surface. The housing 20 should be made of an injection moldable material, such as polyvinyl chloride or acrylic plastic, which will not react with or contaminate the fluid flowing through the device. In the preferred embodiment shown, the housing 20 is made of polyvinyl chloride. A housing 20 made of polyvinyl chloride is somewhat flexible and may assist in damping pressure surges in the fluid caused by pumps or other devices that may be connected to the integrated blood filter and drip chamber device 10. The housing 20 should also be translucent or clear so that the flow of fluids can be observed, particularly when utilizing the device as a drip chamber. A translucent or clear housing 20 will also assist in monitoring the accumulation of contaminates collected by the filter media 62, described below.

In the preferred embodiment shown, the insertion member 40 has an end cap 44 which mates with the open end 26 of the housing to enclose the interior chamber 28. A first port 56, preferably an inlet port comprising a tubing connector, is provided in the end cap 44 for the delivery of fluids into the interior chamber 28. Additional ports 56 may also be provided to accommodate devices such a fluid pressure monitors. In the preferred embodiment shown, a collar 46 is provided on the insertion member 40 adjacent to the end cap 44. As best seen in FIG. 1, the collar 46 fits against the interior surface 30 of the housing 20 when the insertion member 40 is inserted into and mated with the housing 20. A solvent bond is preferably used to seal the joint between the collar 46 and the interior surface 30. A flange 48 prevents the insertion member 40 from being inserted to far into the housing 20.

The insertion member 40 also comprises a filter member 42 which divides the interior chamber 28 into a first volume 12 in fluid communication with the first port 56 and a second volume 14 in fluid communication with the second port 36. The filter member 42 is comprised of a filter media 62 supported by a frame, said frame comprising one or more ribs 50 which are connected to the end cap 44. The filter member 42 has a cylindrical shape which is open at one end and closed at the other end. The open end 52 of the filter member 42 comprises an edge 64 in the shape of a tongue 54. As best seen in FIG. 1, the tongue 54 mates with the groove 32 when the insertion member 40 is inserted into and mated with the housing 20. The tongue 54 and groove 32 joint creates a seal between the edge of the filter member 42 and the interior surface 30 of the housing 20. In the preferred embodiment, the elements of the insertion member 40, other than the filter media 62, are made of an injection moldable material such as acrylic plastic.

Referring to FIG. 3 of the drawings, the ribs 50 are formed on the outside of the filter media 62 to maintain a separation between the filter media 62 and the interior surface 30 of the housing 20. Surges in the fluid pressure may cause the housing 20 to collapse, particularly when the housing is made of a flexible material. The ribs 50 help support the housing 20 and prevent the interior surface 30 from contacting the filter media 62 and inhibiting the flow of fluid through the filter member 42.

Referring to FIG. 4 of the drawings, in an alternative embodiment of a filter 110, the edge 164 of the filter member 142 can be solvent bonded with the interior surface 130 of the housing 120, thereby eliminating the tongue and groove joint (compare FIG. 1).

Referring to FIG. 1 of the drawings, the filter media 62 has a consistency and pore size to allow the passage of fluids to be filtered while preventing the passage of fluid contaminants. In the preferred embodiment, useful for filtering blood to remove blood clots, the filter media has a pore size between about 70 and about 230 microns. The filter media 62 of the preferred embodiment shown comprises a pore size of 170 microns and is made of polyester, such as a PeCap woven fabric supplied by Tetko, Inc.

The closed end 60 of the filter member 42 is spaced apart from the end cap 44 to create a drip chamber 58. The drip chamber 58 can be used to monitor the flow of fluid through the integrated blood filter and drip chamber device 10 by counting the number of drops for a given period of time.

The preferred integrated blood filter and drip chamber device 10 operates as follows. Tubing that will deliver the fluid to be filtered is connected to the first port 56. Tubing that will remove the filtered fluid is connected to the second port 36. Any devices for monitoring the pressure of the fluid are attached to the additional ports 56. Any ports 56 which are not to be used are then plugged. The tubing and the integrated blood filter and drip chamber device 10 are then primed to remove any air within the various components.

If the drip chamber 58 is to utilized, then a sufficient amount of air is to be left inside the interior chamber 28 so that the flow of the fluid through the device 10 can be observed. In addition, the integrated blood filter and drip chamber device 10 must be positioned in a more or less vertical orientation with the first port 56 above the second port 36 so that the fluid will drip freely from the end cap 44.

The fluid to be filtered passes through the first port 56 and enters the first volume 12. The fluid then passes through the filter media 62 and into the second volume 14. The filter media 62 prevents any contaminants within the fluid from passing through the filter media 62 and into the second volume 14. The filtered fluid then exits from the second volume 14 by passing through the second port 36.

The preferred integrated blood filter and drip chamber device 10 is manufactured as follows. The insertion member 40 and the housing 20 are first fabricated in separate operations. The insertion member 40 and the housing 20 are then assembled to form the completed integrated blood filter and drip chamber device 10.

Referring to FIG. 2 of the drawings, the insertion member 40 of the preferred embodiment is formed as a monolithic member supporting the filter media 62. First, the filter media 62 is formed into a sleeve having a tubular or cylindrical shape. This may be accomplished by sealing together two opposing edges of a rectangular piece of filter media. The filter media 62 is then placed over a core pin. The core pin is next inserted into a cavity mold that encloses around the core pin and the filter media 62. The cavities of the mold correspond with the various elements of the insertion element 40 such as the end cap 44, the first port 56, and the frame, including the ribs 50. In a process known as insert injection molding, molten plastic is injected into the cavities of the mold to form the remaining elements of the insertion member 40. The edges of the filter media 62 are sealed during the injection process by embedding plastic into the pores of the filter media. The cavity mold is then opened to remove the core pin and the insertion member 40. Finally, the core pin is removed from the finished insertion member 42.

The housing 20 is formed as a monolithic element. Using an injection molding process, molten polyvinyl chloride is injected into a mold having cavities that correspond to the elements of the preferred housing 20 such as the cylindrical side wall 22, the groove 32 formed by the lip 34, and the second port 36. Following the injection process, the mold is opened and the housing 20 is removed.

Referring to FIG. 1 of the drawings, the assembly of the integrated blood filter and drip chamber device 10 is completed by inserting the insertion member 40 into the housing 20. As the insertion member 40 is inserted into the housing 20, the tongue 54 is inserted into the groove 32. A solvent bond between the collar 46 and the interior surface 30 of the housing 20 seals the interior chamber 28 and completes the assembly of the device 10.

The preferred filter device and method of manufacture described above reduce the number of solvent bonds to no more than two and as few as one. This reduction in the number of solvent bonds greatly reduces the cost of manufacture and improves the integrity of the filter device.

It should be appreciated that the apparatus and methods of the present invention are capable of being incorporated in the form of a variety of embodiments, only a few of which have been illustrated and described above. The invention may be embodied in other forms without departing from its spirit or essential characteristics. For example, other filters beside blood filters may be constructed following this invention. The described embodiments are to be considered in all respects only as illustrative and not restrictive, and the scope of the invention is, therefore, indicated by the appended claims rather than by the foregoing description. All changes which come within the meaning and range of equivalency of the claims are to be embraced within their scope.

## Claims

1. A fluid filter device having a first and a second port, said device comprising:
a) a housing comprising a second port, an interior surface, and a open end;
b) an insertion member comprising;
i) an end cap which mates with the open end of the housing to form an interior chamber within the housing;
ii) a first port on the end cap;
iii) a filter member comprising a filter media supported by a frame, said filter member comprising an edge which sealingly mates with the interior surface of the housing, said filter member dividing the interior chamber into a first volume in fluid communication with the first port and a second volume in fluid communication with the second port;
c) said filter media capable of allowing fluid to flow between the first and second volumes through the filter media while preventing fluid contaminants from passing between the first and second volumes through the filter media.

2. A device according to claim 1, wherein said edge of the filter member is bonded to said interior surface of the housing.

3. A device according to claim 1, wherein said edge of the filter member is mated to said interior surface of the housing with a tongue and groove joint.

4. A device according to claim 1, wherein said interior surface of the housing further comprises a groove formed by a lip which projects inwardly from the interior surface, said filter member further comprises a tongue which mates with the groove.

5. A device according to claim 4, wherein said housing comprises a closed end having a curved surface, said groove is formed extending inwardly from the curved surface.

6. A device according to claim 1, wherein said filter member further comprises a side wall, a closed end, and a open end, said open end being adjacent to the edge.

7. A device according to claim 6, wherein said open end of the filter member is located near the second port of the housing and said closed end of the filter member is spaced apart from the first port of the end cap.

8. A device according to claim 1, wherein said filter media is a blood filter having a pore size of between about 70 and about 230 microns.

9. A device according to claim 1, wherein the end cap, the first port, and the filter frame are formed as a monolithic element.

10. A device according to claim 1, wherein said end cap comprises a plurality of ports.

11. A device according to claim 1, wherein said interior chamber comprises a drip chamber.

12. A device according to claim 1, wherein said first and second ports each comprise a tubing connector.

13. A device according to claim 1, wherein said housing comprises polyvinyl chloride and the elements of said insertion member other than the filter media comprise an injection moldable acrylic plastic.

14. A device according to claim 1, wherein said housing comprises an injection moldable transparent acrylic plastic and the elements of said insertion member other than the filter media comprise an injection moldable acrylic plastic.

15. A fluid filter device comprising:
a) a housing having an interior surface and an interior chamber, said interior chamber comprising a first volume and a second volume, said housing further comprising a groove formed by a lip which projects inwardly from the interior surface;
b) an inlet port to the housing for delivering fluid into the first volume of the interior chamber;
c) an outlet port to the housing for delivering fluid from the second volume of the interior chamber; and
d) a filter member which separates the first volume from the second volume, said filter member capable of allowing fluid to flow from the first volume through the filter member and into the second volume while preventing fluid contaminants from passing from the first volume through the filter member and into the second volume, said filter member further comprising a tongue which mates with the groove in the housing to create a seal between the first volume and the second volume.

16. A device according to claim 15, wherein said filter member comprises a blood filter media having a pore size between about 70 and about 230 microns.

17. A device according to claim 15, wherein said interior chamber comprises a drip chamber.

18. A device according to claim 15, wherein said housing comprises an end cap which supports said filter member.

19. A device according to claim 18, wherein said inlet port is located on said end cap.

20. A device according to claim 19, wherein said end cap comprises a second inlet port.

21. A device according to claim 19, wherein said filter member comprises a frame and a filter media, and said end cap, said inlet port, and said filter frame are formed as a monolithic element.

22. A fluid filter device comprising:
a) a monolithic housing comprising;
i) an outlet port;
ii) an interior surface;
iii) a open end; and
iv) a groove formed by a lip which projects inwardly from the interior surface;
b) a monolithic insertion member comprising;
i) an end cap which mates with the open end of the housing to form an interior chamber within the housing;
ii) an inlet port on the end cap; and
iii) a filter frame supporting a filter media, said filter frame comprising a tongue which mates with the groove on the interior surface of the housing, said filter frame and media dividing the interior chamber into a first volume in fluid communication with the inlet port and a second volume in fluid communication with the outlet port;
c) said inlet port further comprising a tubing connector;
d) said outlet port further comprising a tubing connector; and
e) said filter media capable of allowing fluid to flow from the first volume through the filter media and into the second volume while preventing fluid contaminants from passing from the first volume through the filter media and into the second volume, said filter media having a pore size of between about 70 and about 230 microns.

23. A device according to claim 22, wherein the interior chamber further comprises a drip chamber.

24. A method of manufacturing a filter comprising the steps of:
a) forming a insertion member having an end cap, an inlet port on the end cap, and a filter member having an edge, the filter member comprising a filter frame and a filter media; comprising the steps of:
i) forming the filter media into the shape of a sleeve;
ii) placing the filter media over a core pin;
iii) inserting the core pin into a cavity mold; and
iv) injecting plastic into the cavity mold to form a monolithic member comprising the end cap, inlet port, and the filter frame supporting the filter media;
b) forming by injection molding a monolithic housing having an outlet port, an interior surface, and an open end;
c) assembling the insertion member to the housing by bonding the end cap of the insertion member to the open end of the housing; and
d) sealing the edge of the filter member to the interior surface of the housing with a tongue and groove joint.
